# EUROPEAN PATENT SPECIFICATION

(11) **EP 2 977 368 B1**
(45) Date of publication and mention of the grant of the patent: **12.06.2019**
(21) Application number: 14768179.5
(22) Date of filing: 13.03.2014
(51) Int. Cl.: C07C 209/84, C07C 211/36

(54) **METHOD FOR PRODUCING OPTICALLY ACTIVE TRANS-1,2-DIAMINOCYCLOHEXANE**
VERFAHREN ZUR HERSTELLUNG VON OPTISCH AKTIVEM TRANS-1,2-DIAMINOCYCLOHEXAN
PROCÉDÉ DE PRODUCTION D'UN TRANS-1,2-DIAMINOCYCLOHEXANE OPTIQUEMENT ACTIF

(30) Priority: 19.03.2013 JP 2013056212
(43) Date of publication of application: 27.01.2016
(73) Proprietor: Toray Fine Chemicals Co., Ltd., Tokyo 101-0041 (JP)
(72) Inventor: MORII, Seiji, Nagoya-shi Aichi 455-8502 (JP); MORIMOTO, Masao, Nagoya-shi Aichi 455-8502 (JP)
(74) Representative: Kador & Partner PartG mbB
(86) International application number: PCT/JP2014/056612
(87) International publication number: WO 2014/148344

(56) References cited:
- WO-A1-96/34000
- JP-A- H0 672 903
- JP-A- H07 258 175
- JP-A- 2001 106 656
- JP-A- 2002 316 952
- US-A- 4 085 138
- US-A- 4 085 138
- F. Galsboel: "The Preparation, Separation, and Characterization of the lel3- and ob3-Isomers of Tris(trans-1,2-cyclohexanediamine)rhodium (III) Complexes.'", ACTA CHEMICA SCANDINAVICA, 1 January 1972 (1972-01-01), pages 3605-3611, XP055285833, Retrieved from the Internet: URL:http://actachemscand.org/pdf/acta_vol_ 32a_p0757-0761.pdf [retrieved on 2016-07-05]
- GUANGYOU Z ET AL: "Resolution of beta-aminoalcohols and 1,2-diamines using fractional crystallization of diastereomeric salts of dehydroabietic acid", TETRAHEDRON ASYMMETRY, PERGAMON PRESS LTD, OXFORD, GB, vol. 14, no. 21, 31 October 2003 (2003-10-31), pages 3297-3300, XP004468542, ISSN: 0957-4166, DOI: 10.1016/J.TETASY.2003.08.033
- SCHANZ ET AL: "Improved resolution methods for (R,R)- and (S,S)-cyclohexane-1,2-diamine and (R)- and (S)-BINOL", TETRAHEDRON ASYMMETRY, PERGAMON PRESS LTD, OXFORD, GB, vol. 14, no. 18, 19 September 2003 (2003-09-19), pages 2763-2769, XP005021155, ISSN: 0957-4166, DOI: 10.1016/S0957-4166(03)00586-X
- F. GALSBOEL: 'The Preparation, Separation, and Characterization of the lel3- and ob3-Isomers of Tris(trans-1,2-cyclohexanediamine)rhodium (III) Complexes.' ACTA CHEMICA SCANDINAVICA vol. 26, 1972, pages 3605 - 3611, XP055285833
- THOMAS A.: 'Asymmetric Synthesis via Lithium Chelates, Polyamine-Chelated Alkali Metal Compounds' ADVANCES IN CHEMISTRY vol. 130, 1974, pages 270 - 280, XP002761899
- F Galsboel ET AL: "The Preparation, Separation, and Characterization of the lel3- and ob3-Isomers of Tris(trans-1,2-cyclohexanediamine)rhodium (III) Complexes", Acta Chemica Scandinavia, vol. 26, no. 9, 1 January 1972 (1972-01-01), pages 3605-3611, XP055430006,

## Description

### Technical Field

The present invention relates to a method for producing optically active trans-1,2-diaminocyclohexane, which industrially enables mass production.

### Background Art

As a method for producing optically active trans-1,2-diaminocyclohexane, for example, a method for optically resolving a mixture of trans-1,2-diaminocyclohexane and cis-1,2-diaminocyclohexane with optically active tartaric acid, or a method for optically resolving trans-1,2-diaminocyclohexane with optically active lactic acid is known. As a method for isolating optically active trans-1,2-diaminocyclohexane from a salt of optically active trans-1,2-diaminocyclohexane and an optical resolution agent, a distillation and separation method is known (see Patent Literatures 1 and 2). Further, as a method for isolating optically active trans-1,2-diaminocyclohexane from a salt of optically active trans-1,2-diaminocyclohexane and optically active carboxylic acid, a method in which the salt is reacted with an alkali metal alkoxide in alcohol, the filtrate obtained by filtration is concentrated and then distilled is known (see Patent Literature 3).

When optically active trans-1,2-diaminocyclohexane is produced by these methods, the melting point of the optically active trans-1,2-diaminocyclohexane obtained by distillation is as high as 43 to 45°C, therefore, the taken-out product is solidified at room temperature. For this reason, when being used for weighing, dissolution, moving, or the like, the optically active trans-1,2-diaminocyclohexane is required to be melted by heating, and therefore, the handling has been difficult.

In an isolation method of optically active trans-1,2-diaminocyclohexane by distillation, optically active trans-1,2-diaminocyclohexane that is easy to handle and has favorable nature cannot be obtained by an industrially applicable method. The establishment of the method for obtaining optically active trans-1,2-diaminocyclohexane that is easy to handle and has favorable nature has been desired. Patent Literature 4 discloses a process for the optical resolution of crude trans-1,2-cyclohexanediamine (DACH) by a process using a partial molar quantity of D- or L-tartaric acid in conjunction with a second acid selected from the group consisting of C₁ to C₈ mono- or difunctional carboxylic acids or HCl present at a concentration equivalent to the other amines present in the mixed feedstream being treated, the reaction being run in a substantially aqueous medium as solvent. In this process, a crop of crystals is recovered and added to an excess of aqueous base, the only proviso being that the base chosen liberates DACH from the tartrate salt, typically NaOH is used thereby liberating optically active DACH of about 81-97% or higher optical purity. The optically active DACH product is recovered by extraction of the basic mixture with an organic solvent, preferably benzene, hexane, cyclohexane, xylene, or toluene.

Non-Patent Literature 1 reports the preparation, separation and characterization of isomers of tris-(trans-1,2-cyclohexanediamine)-rhodium (III) complexes. Chiral 1,2-diaminocyclohexane is liberated from a solution with KOH in water. Thus, the amine layer was separated, diluted with ether, sodium was added, left overnight, the amine phase was decanted, treated with active carbon and filtered under nitrogen. The yellowish solution was placed in a water bath and the ether was evaporated by suction until the amine precipitated. The mixture was then cooled to -20 °C before the precipitate was filtered, washed with ether (-20 °C), and dried over potassium hydroxide.

### Citation List

### Patent Literature

Patent Literature 1: Japanese Patent Application Laid-Open (JP-A) No. H07-188121
Patent Literature 2: JP-A No. H07-258175
Patent Literature 3: JP-A No. H11-29535
Patent Literature 4: US Patent No. 4,085,138

### Non-Patent Literature

Non-Patent Literature 1: F. Galsboel et al., Acta Chemica Scandinavia, Vol. 26, No.9, p. 3605-3611 (1972)

### Summary of Invention

### Technical Problem

An object of the present invention is to provide a production method in which optically active trans-1,2-diaminocyclohexane that is easy to handle and has favorable nature can industrially be obtained.

### Solution to Problem

The present invention is a method for producing optically active trans-1,2-diaminocyclohexane, in which a crystal of optically active trans-1,2-diaminocyclohexane is obtained by crystallization from a solution of optically active trans-1,2-diaminocyclohexane, and a solvent of the solution of optically active trans-1,2-diaminocyclohexane is a solvent obtained by adding a solvent having an azeotropic composition with water, and performing azeotropic dehydration, wherein the solvent having an azeotropic composition with water is toluene, or cyclopentyl methyl ether.

### Advantageous Effects of Invention

Optically active trans-1,2-diaminocyclohexane with high quality is obtained in a favorable yield from a solution of optically active trans-1,2-diaminocyclohexane.

The method for producing optically active trans-1,2-diaminocyclohexane of the present invention is easy in handling of weighing, dissolution, moving, and the like as compared with the optically active trans-1,2-diaminocyclohexane obtained by distillation.

According to the present invention, a crystal of optically active trans-1,2-diaminocyclohexane that is easy in handling of weighing, dissolution, moving, and the like can be obtained by the crystallizing of optically active trans-1,2-diaminocyclohexane from a solution of optically active trans-1,2-diaminocyclohexane, and a solvent of the solution of optically active trans-1,2-diaminocyclohexane is a solvent obtained by adding a solvent having an azeotropic composition with water, and performing azeotropic dehydration, wherein the solvent having an azeotropic composition with water is toluene, or cyclopentyl methyl ether.
Further, since a purification effect can also be expected during the crystallization, optically active trans-1,2-diaminocyclohexane with high quality can be obtained.

In the optically active trans-1,2-diaminocyclohexane with high purity, which is produced by a method for producing optically active trans-1,2-diaminocyclohexane of the present invention, a complex with a noble metal such as platinum has a possibility capable of being used as an anticancer agent, and is useful as a raw material for a large number of medicines. Description of Embodiments

Hereinafter, the present invention will be described in detail.

The present invention is a method for producing optically active trans-1,2-diaminocyclohexane, in which a crystal of optically active trans-1,2-diaminocyclohexane is obtained by crystallization from a solution of optically active trans-1,2-diaminocyclohexane, and a solvent of the solution of optically active trans-1,2-diaminocyclohexane is a solvent obtained by adding a solvent having an azeotropic composition with water, and performing azeotropic dehydration, wherein the solvent having an azeotropic composition with water is toluene, or cyclopentyl methyl ether.

In the present invention, examples of the solvent of a solution of optically active trans-1,2-diaminocyclohexane, which is a raw material, include, for example, aromatic hydrocarbons such as benzene, toluene, ethylbenzene, o-xylene, m-xylene, p-xylene, styrene, chlorobenzene, and naphthalene; aliphatic hydrocarbons such as pentane, hexane, heptane, octane, nonane, decane, and dodecane; a halogen-containing solvent such as carbon tetrachloride, dichloromethane, chloroform, and 1,2-dichloroethane; alcohols such as methanol, ethanol, propanol, isopropanol, butanol, isobutanol, sec-butanol, tert-butanol, pentanol, 2-pentanol, hexanol, heptanol, and octanol; aliphatic esters such as allyl acetate, isobutyl acetate, isopropyl acetate, isopentyl acetate, ethyl acetate, vinyl acetate, phenyl acetate, butyl acetate, propyl acetate, benzyl acetate, methyl acetate, isobutyl formate, butyl formate, methyl propionate, ethyl propionate, methyl butyrate, ethyl butyrate, propyl butyrate, isopropyl butyrate, butyl butyrate, isobutyl butyrate, pentyl butyrate, isopentyl butyrate, ethyl isobutyrate, methyl isovalerate, and ethyl isovalerate; aromatic esters such as methyl benzoate, and ethyl benzoate; ethers such as tetrahydrofuran, cyclopentyl methyl ether, diethyl ether, diisopropyl ether, dibutyl ether, anisole, and diphenyl ether; ketones such as acetone, acetylacetone, ethyl methyl ketone, cyclopentanone, cyclohexanone, 3-heptanone, 4-heptanone, 2-pentanone, and 3-pentanone; and nitriles such as acetonitrile. The solvent of a solution of optically active trans-1,2-diaminocyclohexane, which is a raw material, is preferably toluene, ethylbenzene, o-xylene, m-xylene, p-xylene, isopropanol, butanol, isobutanol, sec-butanol, tert-butanol, tetrahydrofuran, cyclopentyl methyl ether, diethyl ether, diisopropyl ether, and ethyl methyl ketone, and more preferably toluene, ethylbenzene, o-xylene, m-xylene, p-xylene, tetrahydrofuran, and cyclopentyl methyl ether. As the solution of optically active trans-1,2-diaminocyclohexane, for example, a solution of optically active trans-1,2-diaminocyclohexane containing water can also be used, and the content of water is preferably 50 times by weight or less to the optically active trans-1,2-diaminocyclohexane. Further, in the solution of optically active trans-1,2-diaminocyclohexane, an alkali salt of carboxylic acid, inorganic alkali, and an inorganic salt may be contained.

A mixture of 1,2-diaminocyclohexane isomers is optically resolved with optically active tartaric acid, and the obtained diastereomer salt of optically active trans-1,2-diaminocyclohexane and optically active tartaric acid is decomposed by sodium hydroxide in a water solvent to obtain a solution containing the optically active trans-1,2-diaminocyclohexane, the obtained solution can be used as a raw material. Further, a diastereomer salt of optically active trans-1,2-diaminocyclohexane and optically active tartaric acid is decomposed by calcium hydroxide in a water solvent to obtain an aqueous solution containing the optically active trans-1,2-diaminocyclohexane, the obtained solution can also be used as a raw material. Alternatively, an massive material of the optically active trans-1,2-diaminocyclohexane isolated by distillation, can also be used as a raw material.

In the method for producing optically active trans-1,2-diaminocyclohexane of the present invention, a solvent of a solution of optically active trans-1,2-diaminocyclohexane is a solvent obtained by adding a solvent having an azeotropic composition with water as defined in claim 1, and performing azeotropic dehydration. In particular, in a case where the raw material is a solution of optically active trans-1,2-diaminocyclohexane containing water, a solvent having an azeotropic composition with water as defined in claim 1 is added, azeotropic dehydration is performed, and the water is removed, then optically active trans-1,2-diaminocyclohexane is separated from the solution as a crystal form by crystallization. At this time, it is more preferred that in order to increase the productivity, the concentration of optically active trans-1,2-diaminocyclohexane is concentrated to 25% by weight or more in advance, and the water is distilled to some extent, then a solvent having an azeotropic composition with water is added, and the azeotropic dehydration is more efficiently performed.

The solvent having an azeotropic composition with water, which is used in the azeotropic dehydration of the present invention is a water-insoluble organic solvent, which is toluene, or cyclopentyl methyl ether. Herein, the water-insoluble organic solvent means an organic solvent having a solubility in water of 10% or less, and preferably 2% or less.

In the azeotropic dehydration of the present invention, a Dean-Stark apparatus is preferably used. By using a Dean stark apparatus, a water layer of the distilled liquid and a water-insoluble organic solvent layer are separated into two layers, therefore, only the water layer can be removed to the outside of the system, and the water-insoluble organic solvent layer can be recycled.

The use amount of the solvent having an azeotropic composition with water, which is used in azeotropic dehydration is preferably 20 times by weight or less to the optically active trans-1,2-diaminocyclohexane in consideration of the productivity.

In the azeotropic dehydration, a Dean stark apparatus is preferably used. When a Dean stark apparatus is used, the production cost can be decreased. In a case where a water-insoluble organic solvent having an azeotropic composition with water is used, the distillate obtained by concentration and azeotropic dehydration is separated into two layers, therefore, an operation, in which only the water layer is removed to the outside of the system, and the water-insoluble organic solvent is returned to the concentrated liquid, is repeated until the distillation of the water layer is no longer performed, and the dehydration can easily be performed. At this time, when the concentration is excessively performed during azeotropic dehydration, the optically active trans-1,2-diaminocyclohexane is gradually distilled, and the yield is decreased, therefore, the concentration of the optically active trans-1,2-diaminocyclohexane in the concentrated liquid during azeotropic dehydration is preferably maintained to 10 to 70% by weight.

The water content in the solution of optically active trans-1,2-diaminocyclohexane in which the water has been removed to the outside of the system, and the solvent has been substituted for a solvent having an azeotropic composition with water, is preferably 2% by weight or less, and more preferably 1% by weight or less. The lower the water content is, the smaller the loss of the optically active trans-1,2-diaminocyclohexane to the crystallization filtrate is during the crystallization, and the yield is improved, therefore, this is preferred.

In the solution of optically active trans-1,2-diaminocyclohexane to which azeotropic dehydration has been performed, in a case where an alkali salt of carboxylic acid, inorganic alkali, and an inorganic salt are contained, these are precipitated, therefore, subjected to solid-liquid separation, and can be removed to the outside of the system.

Examples of the method of crystallization to obtain a crystal of optically active trans-1,2-diaminocyclohexane from a solution of optically active trans-1,2-diaminocyclohexane include, for example, a cooling crystallization method, an evaporation crystallization method, a poor solvent addition crystallization method, a pressure crystallization method, and a reaction crystallization method, preferably cooling crystallization method, an evaporation crystallization method, and a poor solvent addition crystallization method, and more preferably a cooling crystallization method.

In the method for producing optically active trans-1,2-diaminocyclohexane of the present invention, a solvent of a solution of optically active trans-1,2-diaminocyclohexane is preferably a solvent obtained by adding a solvent having an azeotropic composition with water, and performing azeotropic dehydration, and the solvent having an azeotropic composition with water is preferably the same solvent as that used for the crystallization.

Examples of the solvent used for crystallization that is preferably used in the present invention include aromatic hydrocarbons such as benzene, toluene, ethylbenzene, o-xylene, m-xylene, p-xylene, styrene, chlorobenzene, and naphthalene; aliphatic hydrocarbons such as pentane, hexane, heptane, octane, nonane, decane, and dodecane; a halogen-containing solvent such as carbon tetrachloride, dichloromethane, chloroform, and 1,2-dichloroethane; alcohols such as methanol, ethanol, propanol, isopropanol, butanol, isobutanol, sec-butanol, tert-butanol, pentanol, 2-pentanol, hexanol, heptanol, and octanol; aliphatic esters such as allyl acetate, isobutyl acetate, isopropyl acetate, isopentyl acetate, ethyl acetate, vinyl acetate, phenyl acetate, butyl acetate, propyl acetate, benzyl acetate, methyl acetate, isobutyl formate, butyl formate, methyl propionate, ethyl propionate, methyl butyrate, ethyl butyrate, propyl butyrate, isopropyl butyrate, butyl butyrate, isobutyl butyrate, pentyl butyrate, isopentyl butyrate, ethyl isobutyrate, methyl isovalerate, and ethyl isovalerate; aromatic esters such as methyl benzoate, and ethyl benzoate; ethers such as tetrahydrofuran, cyclopentyl methyl ether, diethyl ether, diisopropyl ether, dibutyl ether, anisole, and diphenyl ether; ketones such as acetone, acetylacetone, ethyl methyl ketone, cyclopentanone, cyclohexanone, 3-heptanone, 4-heptanone, 2-pentanone, and 3-pentanone; and nitriles such as acetonitrile. The solvent used for crystallization is more preferably toluene, ethylbenzene, o-xylene, m-xylene, p-xylene, isopropanol, butanol, isobutanol, sec-butanol, tert-butanol, tetrahydrofuran, cyclopentyl methyl ether, diethyl ether, diisopropyl ether, and ethyl methyl ketone, and furthermore preferably toluene, ethylbenzene, o-xylene, m-xylene, p-xylene, tetrahydrofuran, and cyclopentyl methyl ether.

In the solvent used for crystallization, the solubility of the optically active trans-1,2-diaminocyclohexane at the temperature during the separation of the optically active trans-1,2-diaminocyclohexane precipitated by crystallization is preferably 40% by weight or less, and more preferably 30% by weight or less in consideration of the productivity.

Optically active trans-1,2-diaminocyclohexane is crystallized from a solution of optically active trans-1,2-diaminocyclohexane. The optically active trans-1,2-diaminocyclohexane precipitated by crystallization is preferably subjected to solid-liquid separation, as a result of which a crystal of optically active trans-1,2-diaminocyclohexane is obtained. As needed, when the filtrate is concentrated to adjust the concentration of optically active trans-1,2-diaminocyclohexane, and the recrystallization is performed, a crystal of optically active trans-1,2-diaminocyclohexane can further be obtained. When the optically active trans-1,2-diaminocyclohexane obtained by the first crystallization is combined, the optically active trans-1,2-diaminocyclohexane can be obtained in a high yield. The filtrate can be recycled as a solvent, and again can also be used as a solvent during the crystallization of optically active trans-1,2-diaminocyclohexane.

The thus obtained crystal of optically active trans-1,2-diaminocyclohexane can be the optically active trans-1,2-diaminocyclohexane that has higher purity than that of the optically active trans-1,2-diaminocyclohexane in a raw material. In a case where cis-1,2-diaminocyclohexane that is a related impurity, and trans-1,2-diaminocyclohexane that is an optical isomer are contained, the removal by distillation has been difficult, however, according to the method of the present invention, cis-1,2-diaminocyclohexane that is a related substance, and trans-1,2-diaminocyclohexane that is an optical isomer can also be removed. Further, the optically active trans-1,2-diaminocyclohexane isolated by distillation is solidified and becomes a massive material, therefore, the handling has been difficult, however, the crystal of optically active trans-1,2-diaminocyclohexane obtained in the present invention has flowability, and the operations of weighing, dissolution, and moving are easily performed.

Hereinafter, an example of the method for producing optically active trans-1,2-diaminocyclohexane using toluene, or cyclopentyl methyl ether is shown.

At first, a solution of optically active trans-1,2-diaminocyclohexane that is a raw material is charged. The concentration of optically active trans-1,2-diaminocyclohexane is concentrated to 25% by weight or more. This operation can be omitted in a case where the concentration of optically active trans-1,2-diaminocyclohexane is 25% by weight or more. Next, into the solution, toluene, or cyclopentyl methyl ether is added, and the resultant mixture is subjected to azeotropic dehydration using a Dean stark apparatus. The pressure in the reaction system during the azeotropic dehydration is adjusted to preferably normal pressure to 16,9 kPa (50 Torr) and more preferably normal pressure to 33,9 kPa (100 Torr). The temperature in the reaction system during the azeotropic dehydration is preferably 40 to 110°C, and more preferably 50 to 100°C. Only the water layer of the distilled liquid is removed to the outside of the system, and the azeotropic dehydration is performed until the distillation of the water is no longer performed. In a case where an alkali salt of carboxylic acid, inorganic alkali, an inorganic salt are precipitated in the solution of optically active trans-1,2-diaminocyclohexane to which dehydration has been performed, the precipitated alkali salt of carboxylic acid, inorganic alkali, and inorganic salt are cooled, then subjected to solid-liquid separation, and removed to the outside of the system. The toluene or cyclopentyl methyl ether solution of the optically active trans-1,2-diaminocyclohexane from which water has been removed is concentrated to adjust the concentration of 1,2-diaminocyclohexane so as to be 30 to 60%. The temperature at which a seed crystal of optically active trans-1,2-diaminocyclohexane is added is different depending on the concentration of the optically active trans-1,2-diaminocyclohexane, however, is generally lower than the melting point of optically active 1,2-diaminocyclohexane, and 40°C or less, and more preferably 15 to 30°C. A slurry solution in which a crystal of optically active trans-1,2-diaminocyclohexane has been precipitated is further cooled. The cooling temperature before the solid-liquid separation is preferably -20 to 10°C, and more preferably -15 to 5°C. In order to obtain a crystal of optically active trans-1,2-diaminocyclohexane in a stable yield, the aging time is required to be sufficiently taken at the cooling temperature before the solid-liquid separation. In general, the time is 0.5 to 24 hours, and more preferably 1 to 20 hours. The slurry solution is subjected to solid-liquid separation using a centrifugal filter, a pressure filter, or the like, and dried, as a result of which a crystal of optically active trans-1,2-diaminocyclohexane is obtained.

### Examples

Hereinafter, the present invention will be described in more detail by way of Examples. Further, the chemical purity, and the optical purity of optically active trans-1,2-diaminocyclohexane were measured by the method shown below.

### <Chemical purity analysis method>

Around 0.1 g of trans-1,2-diaminocyclohexane was collected in a 10 mL measuring flask, and to which methanol was put up to the marked line for the dilution. The prepared solution was analyzed by gas chromatography, and the purity was determined by area percentage.

### Gas chromatography analysis conditions

| | |
|---|---|
| Column: | WACOTT Fused Silica |

CP-Sil-5CB, ID 0.32 mm, Film 5 µm, Length 60 m

| | |
|---|---|
| He flow rate: | 50 mL/min |
| Column temperature: | 160°C |
| Injection temperature: | 200°C |
| Detector temperature: | 200°C |
| Detector: | FID |
| Injection volume: | 1.0 µL |
| Split ratio: | 61 |

**Retention time**

| | |
|---|---|
| Trans-1,2-diaminocyclohexane: | 21.0 minutes |
| Cis-1,2-diaminocyclohexane: | 23.7 minutes |

### <Optical purity analysis method>

Around 0.25 g of trans-1,2-diaminocyclohexane was accurately weighed and put into a 20 mL measuring flask, and to which acetonitrile was put up to the marked line for the dilution. 1 mL of the solution was collected in a 10 mL measuring flask, and to which acetonitrile was put up to the marked line for the dilution. 0.2 mL of trans-1,2-diaminocyclohexane solution was collected in a 1.5 mL sample bottle, and to which 0.8 mL of 1.0% GITC acetonitrile solution was added, then the mixture was reacted at room temperature for 10 minutes. After the reaction, 0.2 mL of acetonitrile solution of 1.0% monoethanolamine was added, and then the mixture was further reacted at room temperature for 3 minutes. After the reaction, 0.2 mL of 5% phosphoric acid aqueous solution was added, and a sample solution was obtained. 10 µL of the sample solution was injected into high performance liquid chromatography, and the optical purity was determined by area ratio. (Herein, GITC means 2, 3, 4, 6-tetra-o-acetyl-β-D-glucopyranosyl isothiocyanate).

### High performance liquid chromatography analysis condition

| | |
|---|---|
| Column: | Mightysil RP-18GP, 4.6 mm × 15 cm (5 µm) (KANTO CHEMICAL CO., INC.) |
| Mobile phase: | 0.05% phosphoric acid / acetonitrile = 55 / 45 (v/v) |
| Flow rate: | 1.0 mL/min |
| Temperature: | 40°C |
| Detector: | UV (254 nm) |

**Retention time**

| | |
|---|---|
| A GITC derivatized compound of (1S,2S)-trans-1,2-diaminocyclohexane (hereinafter, abbreviated as S-1,2-diaminocyclohexane): | 8.3 minutes |
| A GITC derivatized compound of cis-1,2-diaminocyclohexane: | 10.5 minutes |
| A GITC derivatized compound of (1R,2R)-trans-1,2-diaminocyclohexane (hereinafter, abbreviated as R-1,2-diaminocyclohexane)R-1,2-diaminocyclohexane: | 12.1 minutes |

### (Reference Example 1)

Into a 5000 mL four-neck flask equipped with a thermometer, a dropping funnel, and a stirrer, 330.2 g (2.2 mol) of L-tartaric acid, and 963.6 g of water were charged. Next, while stirring the mixture, 808.7 g (7.1 mol) of a mixture of 1,2-diaminocyclohexane isomers, in which the ratio of cis-1,2-diaminocyclohexane and trans-1,2-diaminocyclohexane is 1.2 : 2, that is, an optical isomer ratio of a trans-isomer (R-isomer / S-isomer = 1 / 1), was added. While being kept at 60°C, the reaction mixture was stirred for one hour, and then 586.5 g (9.8 mol) of acetic acid was added dropwise from a dropping funnel in 1.0 hour. After the completion of the dropwise addition, the mixture was further heated and stirred at 90°C for two hours, and then cooled to 15°C over five hours or more. After the mixture was stirred at 15°C for three hours, the mixture was subjected to solid-liquid separation by a centrifugal dehydrator, then rinsed with water, and dried to obtain 446.8 g (1.7 mol) of R-1,2-diaminocyclohexane. L-tartaric acid. The yield was 75% for R-1,2-diaminocyclohexane. Further, as a result of analysis of a diastereomer salt, the optical purity was 97.3% e.e, and the trans/cis ratio was 29. The obtained crystal was suspended in 1269.0 g of water, further, into which 195.6 g (2.6 mol) of calcium hydroxide was added, then the mixture was heated up to 80°C, and aged at the same temperature for five hours. The resultant mixture was cooled to 25°C over two hours, the precipitated crystal was separated by filtration, and 694.8 g of L-tartaric acid calcium salt was removed. 1756.3 g of filtrate was obtained, and 187.4 g of the R-1,2-diaminocyclohexane separated from L-tartaric acid was present in the filtrate. Yield: 94%.

### (Example 1)

Into a 1000 mL four-neck flask equipped with a thermometer, a condenser, and a stirrer, 798.3 g of a filtrate containing 85.2 g of R-1,2-diaminocyclohexane (0.8 mol, Quality: the chemical purity was 96.6%, the optical purity was 97.3% e.e., and the trans/cis ratio was 29) obtained in Reference Example 1 was charged, concentrated under reduced pressure, and 601.5 g of water was distilled off. Next, 446.6 g of toluene was added into the concentrated liquid, in the mixed solution, water was azeotroped with toluene at normal pressure under the condition of 84 to 87°C using a Dean stark apparatus. Only the water layer in the distillate was removed, and the azeotropic dehydration was continued until the water layer in the solution in the reactor was no longer distillated. After cooling, filter filtration was performed, and insolubles in the solution were removed. The obtained filtrate was concentrated under reduced pressure, and 139 g of a concentrated liquid was obtained. The concentrated liquid was cooled to 20°C, 0.01 g of R-1,2-diaminocyclohexane was added as a seed crystal to precipitate a crystal, and the precipitated crystal was aged at 20°C for one hour. The resultant was cooled to 0 to 5°C over two hours, and aged at 0 to 5°C for two hours. The precipitated crystal was subjected to solid-liquid separation by a centrifugal dehydrator, and dried to obtain 53.4 g of R-1,2-diaminocyclohexane in a crystal form. (Yield: 63%) The obtained crystal form was a flowable needle crystal. Part of the crystal was sampled, and the sample was subjected to the analysis evaluation of R-1,2-diaminocyclohexane. The quality of R-1,2-diaminocyclohexane was a chemical purity of 100%, an optical purity of 99.9% e.e, and a trans/cis ratio of 894. On the other hand, the filtrate had 84.5% e.e, and a trans/cis ratio of 8.4.

### (Comparative Example 1)

Into a 1000 mL four-neck flask equipped with a thermometer, a condenser, and a stirrer, 798.3 g of a filtrate containing 85.2 g of R-1,2-diaminocyclohexane (0.8 mol, Quality: the chemical purity was 96.6%, the optical purity was 97.3% e.e., and the trans/cis ratio was 29) obtained in Reference Example 1 was charged, concentrated under reduced pressure, and 656.2 g of water was distilled off. Next, 353.6 g of 2-propanol was added into the concentrated liquid, the mixed solution was heated, and concentrated at normal pressure under the condition of 100°C, then 2-propanol and water were distilled off to obtain 142.7 g of a crude material of R-1,2-diaminocyclohexane. The crude material was subjected to simple distillation under the conditions of 80°C, and 20 mmHg (2.6 kPa), 79.9g of an initial distillate was cut off, and then 49.4 g (0.4 mol) of a main distillate of R-1,2-diaminocyclohexanewas obtained. (Yield: 58%) The obtained main distillate of R-1,2-diaminocyclohexane was solidified and in a massive state. The massive material of R-1,2-diaminocyclohexane was completely melted, and sampled. The sample was subjected to the analysis evaluation of R-1,2-diaminocyclohexane. The quality of R-1,2-diaminocyclohexane was a chemical purity of 96.7% (cis-isomer 3.3%), an optical purity of 97.3% e.e, and a trans/cis ratio of 29.

### (Reference Example 2)

Into a 1000 mL four-neck flask equipped with a thermometer, a dropping funnel, and a stirrer, 98.6 g (0.7 mol) of D-tartaric acid, and 259.6 g of water were charged. Next, while stirring the mixture, 225.3 g (2.0 mol) of a mixture, in which the ratio of cis-1,2-diaminocyclohexane and trans-1,2-diaminocyclohexane is 1 : 2, that is, an optical isomer ratio of a trans-isomer (R-isomer / S-isomer = 1 / 1), was added. While being kept at 60°C, the reaction mixture was stirred for one hour, and then 158.0 g (2.6 mol) of acetic acid was added dropwise from a dropping funnel in 1.0 hour. After the completion of the dropwise addition, the mixture was further heated and stirred at 90°C for two hours, and then cooled to 15°C over five hours or more. After being stirred at 15°C for three hours, the mixture was subjected to solid-liquid separation by a centrifugal dehydrator, then rinsed with water, and dried to obtain 144.5 g (0.5 mol) of S-1,2-diaminocyclohexane D-tartaric acid. The yield was 83% for S-1,2-diaminocyclohexane. Further, as a result of analysis of the S-1,2-diaminocyclohexane contained in a diastereomer salt, the optical purity was 97.2% e.e, and the trans/cis ratio was 40. The obtained crystal was suspended in 1054.2 g of water, heated up to 100°C, and aged for four hours, then the resultant was gradually cooled, subjected to solid-liquid separation at 5°C, and rinsed with water, as a result of which, 115.9 g of a crystal containing solution was obtained. The yield was 78%, the optical purity was 99.6%, and a cis-isomer was not detected. Into the obtained crystal, 177.3 g of a 25% aqueous sodium hydroxide solution was added, the salt was decomposed while being kept at 70 to 80°C, and 88.0 g of the upper layer obtained after the liquid separation was separated. In the upper layer, 47.7 g of the S-1,2-diaminocyclohexane separated from D-tartaric acid was present, and the water content was 37.8% (33.3 g). Yield: 98%.

### (Example 2)

Into a 200 mL four-neck flask equipped with a thermometer, a condenser, and a stirrer, 40.0 g of a upper layer containing 21.7 g of the S-1,2-diaminocyclohexane (0.19 mol, Quality: the chemical purity was 99.9%, the optical purity was 99.3% e.e., a cis-isomer was not detected, and a trans-isomer was only detected) obtained by liquid separation in Reference Example 2, and 108.5 g of toluene were charged, and mixed. In the mixed solution, water was azeotroped with toluene at 84 to 87°C under normal pressure using a Dean stark apparatus. Only a water layer in the distillate was removed, and the azeotropic dehydration was continued until the distillation of the water layer in the solution in the reactor was no longer distillated. After cooling, the precipitated D-tartaric acid disodium salt was subjected to solid-liquid separation by using a centrifugal dehydrator, and removed. Next, in the filtrate, toluene was distilled off under reduced pressure. The concentrated liquid was cooled to 20°C, 0.01 g of S-1,2-diaminocyclohexane was added as a seed crystal to precipitate a crystal, and the precipitated crystal was aged at 20°C for one hour. The resultant was cooled to 0 to 5°C over two hours, and aged at 0 to 5°C for two hours. The precipitated crystal was subjected to solid-liquid separation by a centrifugal dehydrator, and dried to obtain 16.3 g (0.14 mol) of S-1,2-diaminocyclohexane in a crystal form. (Yield: 75%) The obtained crystal form was a flowable needle crystal. Part of the crystal was sampled, and the sample was subjected to the analysis evaluation of S-1,2-diaminocyclohexane. The quality of S-1,2-diaminocyclohexane was a chemical purity of 100%, an optical purity of 100% e.e, and a cis-isomer was not detected.

### (Comparative Example 2)

Into a 200 mL four-neck flask equipped with a thermometer, a condenser, and a stirrer, 40.0 g of a upper layer containing 21.7 g of the S-1,2-diaminocyclohexane (0.19 mol, Quality: the chemical purity was 99.9%, the optical purity was 99.6% e.e., a cis-isomer was not detected, and a trans-isomer was only detected) obtained by liquid separation in Reference Example 2, and 145.6 g of 2-propanol were charged, and aged for 30 minutes while being stirred. Next, the precipitated D-tartaric acid disodium salt was filtered, then the filtrate was concentrated and distilled, and 13.3 g (0.12 mol) of S-1,2-diaminocyclohexane was obtained. (Yield: 61%) The obtained S-1,2-diaminocyclohexane was solidified and in a massive state. The massive material of S-1,2-diaminocyclohexane was completely melted, and sampled. The sample was subjected to the analysis evaluation of S-1,2-diaminocyclohexane. The quality of S-1,2-diaminocyclohexane was a chemical purity of 99.6%, an optical purity of 99.6% e.e, and a trans/cis ratio was 999.

### (Example 3)

Into a 1 L four-neck flask attached with a thermometer, a vacuum stirrer, and a condenser, 212.4 g of melted R-1,2-diaminocyclohexane (1.9 mol, Quality: the chemical purity was 9.99%, the optical purity was 99.7% e.e., and the trans/cis ratio was 999) was charged. Next, 424.7 g of toluene (2.0 wt times / R-1,2-diaminocyclohexane) was added, and the mixture was stirred, and dissolved. The resultant solution was cooled to 13 to 15°C, and then 0.01 g of R-1,2-diaminocyclohexane was added as a seed crystal to precipitate a crystal, and the precipitated crystal was aged at 13 to 15°C for one hour. The resultant was cooled to 0 to 5°C over two hours, and aged at 0 to 5°C for three hours. The precipitated crystal was subjected to solid-liquid separation by a centrifugal dehydrator, and dried to obtain 133.1 g of R-1,2-diaminocyclohexane in a crystal form (yield: 63%). The obtained crystal form was a flowable needle crystal. Part of the crystal was sampled, and the sample was subjected to the analysis evaluation of R-1,2-diaminocyclohexane. The quality of R-1,2-diaminocyclohexane was a chemical purity of 100%, and an optical purity of 100% e.e, and cis-1,2-diaminocyclohexane was not detected. 480.1 g of solid-liquid separated filtrate (R-1,2-diaminocyclohexane having a concentration of 16.5%, 79.3 g) was concentrated under reduced pressure, and 319.8 g was distilled off. The concentrated liquid was cooled to 23 to 25°C, 0.01 g of R-1,2-diaminocyclohexane was added as a seed crystal to precipitate a crystal, and the precipitated crystal was aged at 23 to 15°C for one hour. The resultant was cooled to 0 to 5°C over two hours, and aged at 0 to 5°C for three hours. The precipitated crystal was subjected to solid-liquid separation by a centrifugal dehydrator, and dried to obtain 52.6 g of R-1,2-diaminocyclohexane in a crystal form (yield: 73%) . The obtained crystal form was a flowable needle crystal. Part of the crystal was sampled, and the sample was subjected to the analysis evaluation of R-1,2-diaminocyclohexane. The quality of R-1,2-diaminocyclohexane was a chemical purity of 100%, an optical purity of 100% e.e, and cis-1,2-diaminocyclohexane was not detected. The R-1,2-diaminocyclohexane in a crystal form, which had been recovered by two times of crystallization, was 185.6 g, and the yield was 87%.

### (Example 4)

Into a 1 L four-neck flask attached with a thermometer, a vacuum stirrer, and a condenser, 285.3 g of melted R-1,2-diaminocyclohexane (2.5 mol, Quality: the chemical purity was 99.9%, the optical purity was 99.7% e.e., and the trans/cis ratio was 999) was charged. Next, 285.3 g of cyclopentyl methyl ether (1.0 wt time / R-1,2-diaminocyclohexane) was added, and the mixture was stirred, and dissolved. The resultant solution was cooled to 22 to 24°C, and then 0.01 g of R-1,2-diaminocyclohexane was added as a seed crystal to precipitate a crystal, and the precipitated crystal was aged at 22 to 24°C for one hour. The resultant was cooled to 0 to 5°C over two hours, and aged at 0 to 5°C for 15 hours. The precipitated crystal was subjected to solid-liquid separation by a centrifugal dehydrator, and dried to obtain 219.6 g of R-1,2-diaminocyclohexane in a crystal form (yield: 77%). The obtained crystal form was a flowable needle crystal. Part of the crystal was sampled, and the sample was subjected to the analysis evaluation of R-1,2-diaminocyclohexane. The quality of R-1,2-diaminocyclohexane was a chemical purity of 100%, an optical purity of 100% e.e, and cis-1,2-diaminocyclohexane was not detected. 312.8 g of solid-liquid separated filtrate (R-1,2-diaminocyclohexane having a concentration of 21.0%, 65.8 g) was concentrated under reduced pressure, and 175.7 g was distilled off. The concentrated liquid was cooled to 19 to 21°C, 0.01 g of R-1,2-diaminocyclohexane was added as a seed crystal to precipitate a crystal, and the precipitate crystal was aged at 19 to 21°C for one hour. The resultant was cooled to 0 to 5°C over two hours, and aged at 0 to 5°C for 15 hours. The precipitated crystal was subjected to solid-liquid separation by a centrifugal dehydrator, and dried to obtain 35.2 g of R-1,2-diaminocyclohexane in a crystal form (yield: 55%). The obtained crystal form was a flowable needle crystal. Part of the crystal was sampled, and the sample was subjected to the analysis evaluation of R-1,2-diaminocyclohexane. The quality of R-1,2-diaminocyclohexane was a chemical purity of 100%, an optical purity of 100% e.e, and cis-1,2-diaminocyclohexane was not detected. The R-1,2-diaminocyclohexane in a crystal form, which had been recovered by two times of crystallization, was 254.8 g, and the yield was 89%.

### (Comparative Example 3)

Into a 100 mL four-neck flask attached with a thermometer, a condenser, and a stirrer, 11.4 g of melted R-1,2-diaminocyclohexane (0.1 mol, Quality: the chemical purity was 99.9%, the optical purity was 99.7% e.e., and the trans/cis ratio was 999) was charged, and into which 100.0 g of dichloromethane was added, and the dichloromethane was concentrated at normal pressure under the condition of 40°C until being crystallized. At a time when 95 g of dichloromethane was distilled off, the concentrated liquid was solidified at once, and the stirring became poor, as a result of which, the crystallization cannot be performed.

### Industrial Applicability

In the method for producing optically active trans-1,2-diaminocyclohexane of the present invention, optically active trans-1,2-diaminocyclohexane with high purity can be obtained from a solution of optically active trans-1,2-diaminocyclohexane in a favorable yield.

In the optically active trans-1,2-diaminocyclohexane with high purity, which is produced by a method for producing optically active trans-1,2-diaminocyclohexane of the present invention, a complex with a noble metal such as platinum has a possibility capable of being used as an anticancer agent, and is useful as a raw material for a large number of medicines.

## Claims

1. A method for producing optically active trans-1,2-diaminocyclohexane, wherein
a crystal of optically active trans-1,2-diaminocyclohexane is obtained by crystallization from a solution of optically active trans-1,2-diaminocyclohexane, and
a solvent of the solution of optically active trans-1,2-diaminocyclohexane is a solvent obtained by adding a solvent having an azeotropic composition with water, and performing azeotropic dehydration, wherein the solvent having an azeotropic composition with water is toluene, or cyclopentyl methyl ether.

2. The method for producing optically active trans-1,2-diaminocyclohexane according to Claim 1, wherein
the solvent having an azeotropic composition with water is the same solvent as that used for crystallization.

## Patentansprüche

1. Verfahren zur Herstellung von optisch aktivem trans-1,2-Diaminocyclohexan, wobei
ein Kristall aus optisch aktivem trans-1,2-Diaminocyclohexan durch Kristallisation aus einer Lösung von optisch aktivem trans-1,2-Diaminocyclohexan erhalten wird, und
ein Lösungsmittel der Lösung von optisch aktivem trans-1,2-Diaminocyclohexan ein Lösungsmittel ist, das durch Zugaben eines Lösungsmittels, das eine azeotrope Zusammensetzung mit Wasser aufweist, und durch Durchführen einer azeotropen Dehydrierung erhalten wird, wobei das Lösungsmittel, das eine azeotrope Zusammensetzung mit Wasser aufweist, Toluol oder Cyclopentylmethylether ist.

2. Verfahren zur Herstellung von optisch aktivem trans-1,2-Diaminocyclohexan nach Anspruch 1, wobei
das Lösungsmittel , das eine azeotrope Zusammensetzung mit Wasser aufweist, das gleiche Lösungsmittel ist, wie das, das für die Kristallisation verwendet wird.

## Revendications

1. Procédé pour produire du trans-1,2-diaminocyclohexane optiquement actif, dans lequel
un cristal de trans-1,2-diaminocyclohexane optiquement actif est obtenu par cristallisation à partir d'une solution de trans-1,2-diaminocyclohexane optiquement actif, et
un solvant de la solution de trans-1,2-diaminocyclohexane optiquement actif est un solvant obtenu par addition d'un solvant ayant une composition azéotropique avec l'eau, et mise en oeuvre d'une déshydratation azéotropique, où le solvant ayant une composition azéotropique avec l'eau est le toluène ou le cyclopentylméthyléther.

2. Procédé pour produire du trans-1,2-diaminocyclohexane optiquement actif selon la revendication 1, dans lequel
le solvant ayant une composition azéotropique avec l'eau est le même solvant que celui utilisé pour la cristallisation.
